# EUROPÄISCHE PATENTSCHRIFT

(11) **EP 1 044 712 B1**
(45) Veröffentlichungstag und Bekanntmachung des Hinweises auf die Patenterteilung: **15.09.2004**
(21) Anmeldenummer: 00106980.6
(22) Anmeldetag: 01.04.2000
(51) Int. Cl.: B01D 19/04, C10M 171/00

(54) **Entschäumer zur Entschäumung wässriger Medien**
Defoamer for defoaming of aqueous media
Agent antimousse pour milieux aqueux

(30) Priorität: 16.04.1999 DE 19917186
(43) Veröffentlichungstag der Anmeldung: 18.10.2000
(73) Patentinhaber: Goldschmidt AG, 45127 Essen (DE)
(72) Erfinder: Ebbrecht, Thomas, Dr., 44892 Bochum (DE); Gippert, Michael, 45138 Essen (DE); Hartmann, Martina, 47167 Duisburg (DE); Josten, Wolfgang, Dr., 53639 Königswinter (DE); Muss, Peter, 45359 Essen (DE); Silber, Stefan, Dr., 47804 Krefeld (DE); Sucker, Roland, 59368 Werne (DE)

(56) Entgegenhaltungen:
- EP-A- 0 115 585
- DE-A- 2 557 898
- DE-A- 2 558 040

## Beschreibung

Die Erfindung betrifft Entschäumer zur Entschäumung wäßriger Medien, die als die Entschäumung entscheidend beeinflussenden hydrophoben Festkörper bestimmte Harnstoffderivate enthalten, welche durch Kristallisation aus einer klaren, homogen im Trägermedium verteilten Schmelze heraus die für ihre besondere Wirksamkeit als Entschäumer erforderlichen Eigenschaften erhalten.

In vielen technischen Verfahren, insbesondere, wenn in wäßrigen Medien gearbeitet wird, ist es notwendig, die unerwünschte Bildung von Schaum während der Herstellungs- oder Verarbeitungsprozesse zurückzudrängen oder ganz zu verhindern. Dies kann erreicht werden, indem man sogenannte Antischaummittel oder Entschäumer hinzugibt, die schon bei sehr geringen Einsatzkonzentrationen ab etwa 0,001 Gew.-% in der Lage sind, unerwünschte Schäume zu vermeiden oder zu zerstören. Solche dem Stand der Technik gemäßen Entschäumer sind zum Beispiel Silikonöle, Mineralöle, hydrophobe Polyoxyalkylene, langkettige Alkohole sowie Mischungen dieser Produkte untereinander und Emulsionen daraus. Zur Verstärkung der Wirksamkeit werden häufig noch sogenannte hydrophobe Festkörper in Mengen von 0,1 bis 10 Gew.-% zugegeben, die gezielt Entnetzungsvorgänge an Schaumlamellen fördern und somit den Schaumzusammenbruch sehr wirksam unterstützen. Geeignete hydrophobe Festkörper sind entsprechende Kieselsäuren, Metallstearate, Polyolefine und Wachse.

Auch die Verwendung von Harnstoff und Harnstoffderivaten als Zusatz zu Entschäumerformulierungen ist an sich bekannt.

In EP-A-0 115 585 werden Harnstoffe beschrieben, welche in situ in einem organischen Trägermedium bei verhältnismäßig niedrigen Temperaturen hergestellt werden und entschäumende Eigenschaften für wäßrige Medien aufweisen. Diese erhält man durch Zusammengeben vorzugsweise äquivalenter Mengen von Isocyanaten und Aminen in dem entsprechenden organischen Trägermedium bei Temperaturen unterhalb des Schmelzpunktes des Reaktionsproduktes.

Dabei werden Harnstoffderivate der allgemeinen Formel 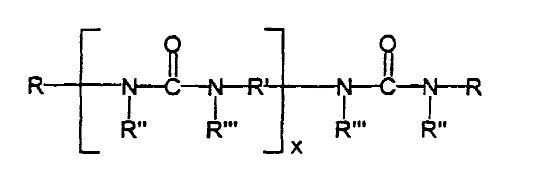
- R =: Alkyl C₄-C₃₀
- R' =: einfache chemische Bindung; Alkylen C₂-C₁₂, ein- bis zweikernige Arylreste, die am Arylrest zusätzliche Alkylgruppen C₁-C₉ aufweisen, Cycloalkylen
- R'' =: H, Alkyl C₁-C₂₄
- R''' =: H, -CH₃-
- x =: 0-5
erhalten.

Es wird ausdrücklich betont, daß die Harnstoffe beim Erhitzen über Ihren Schmelzpunkt hinaus, oder wenn die Harnstoffe oberhalb ihres Schmelzpunktes hergestellt werden, nur noch eine unwesentliche entschäumende Wirkung besitzen. Dies wird in Zusammenhang gebracht mit der Ausbildung monodisperser oder mizellarer Strukturen bei der In-situ-Bildung der Harnstoffderivate.

Nachteilig dabei ist, daß man durch die In-situ-Bildung der Harnstoffderivate gemäß der in EP-A-0 115 585 beschriebenen Verfahrensweise auf Trägermedien angewiesen ist, die nicht mit Aminen und insbesondere auch nicht mit Isocyanaten reagieren können. So scheiden zum Beispiel die als Trägermedien für Entschäumer dem Fachmann wohlbekannten hydroxyfunktionellen Polyoxypropylene und auch hydroxyfunktionellen Polyoxyalkylenpolysiloxane eben ob ihrer Hydroxyfunktionalität aus. Prinzipiell ist zwar die Reaktion eines Amins mit einem Isocyanat gegenüber der Reaktion einer Hydroxyverbindung mit einem Isocyanat mit Bezug auf die Reaktionsgeschwindigkeit eindeutig bevorzugt, doch reagieren auch Hydroxyverbindungen gerade in Gegenwart der als Katalysatoren für die nukleophile Addition von Hydroxyverbindungen an Isocyanaten bekannten Amine zu unkontrollierbaren Anteilen mit ein und führen so zu, wenn überhaupt, nur erschwert kristallisierenden Harnstoffderivaten. Dies hätte natürlich direkten Einfluß auf die Wirksamkeit der gebildeten Harnstoffderivate und würde damit zumindest zu nicht oder kaum reproduzierbaren Ergebnissen beim Einsatz als Entschäumer führen.

Überraschenderweise wurde nun gefunden, daß zur Herstellung von Entschäumerformulierungen mit Harnstoffderivaten als hydrophobem Festkörper die oben beschriebene In-situ-Herstellung solcher Harnstoffderivate unterhalb ihres Schmelzpunktes weder nötig noch vorteilhaft ist, sondern sogar durch gezielte Schmelz- und Rekristallisationsvorgänge Entschäumerformulierungen mit verbesserten Eigenschaften herzustellen sind, die eben durch die methodische Variation von Schmelz- und Rekristallisationsbedingungen maßgeschneiderte Eigenschaftsprofile ermöglichen. Außerdem kann man bei Bedarf, durch separate Herstellung der Harnstoffderivate, auch ausschließlich Trägermedien verwenden, die ob ihrer potentiellen Reaktivität gegenüber Aminen und/oder Isocyanaten wegen der oben beschriebenen Nebenreaktionen keine Fällungskristallisation auf dem in EP-A-0 115 585 beschrieben Wege zulassen. Dazu zählen zum Beispiel hydrophobe Polyoxyalkylene und organomodifizierte Siloxane, die auch noch Hydroxyfunktionen enthalten können.

Dies gelingt gerade auch mit Harnstoffderivaten, die in EP-A-0 115 585 explizit beschrieben werden und die somit nach bisherigem Stand der Technik bei dem in diesem Patent beschriebenen Verfahren keine außergewöhnliche Wirksamkeit in Entschäumerformulierungen mehr zeigen sollten. Solche Harnstoffderivate sind leicht aus den entsprechenden Isocyanaten und Aminen herstellbar.

Gegenstand der Erfindung sind somit Mittel zur Entschäumung wäßriger Medien, die als Entschäumer Harnstoffderivate der Formel I 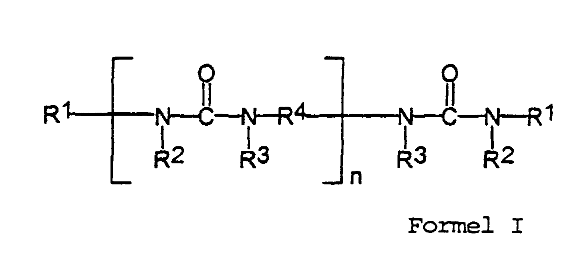 wobei
R¹ - ein Kohlenwasserstoffrest mit 4 bis 30 Kohlenstoffatomen oder ein Kohlenwasserstoffrest mit 4 bis 24 Kohlenstoffatomen und einem Stickstoffatom oder ein Kohlenwasserstoffrest mit 4 bis 30 Kohlenstoffatomen und einer Carbonylgruppe,
R² - ein Wasserstoffatom oder ein Kohlenwasserstoffrest mit 1 bis 24 Kohlenstoffatomen,
R³ - ein Wasserstoffatom oder ein Kohlenwasserstoffrest mit 1 bis 24 Kohlenstoffatomen,
R⁴ - ein organischer Rest mit 2 bis 30 Kohlenstoffatomen und
n - 0 bis 5 sind, in Form von Feststoffpartikeln enthalten,
welche durch Kristallisation aus einer in einem Trägermedium verteilten klaren, homogenen Schmelze heraus erhalten werden.

Bevorzugt sind solche Mittel, bei denen R¹ ein Kohlenwasserstoffrest mit 4 bis 24 Kohlenstoffatomen ist, R² ein Wasserstoffatom, R³ ein Wasserstoffatom und R⁴ ein Kohlenwasserstoffrest mit 2 bis 24 Kohlenstoffatomen sind.

Es ist also somit für die vorliegende Erfindung völlig unerheblich, ob die erfindungsgemäßen Harnstoffderivate separat hergestellt und als Feststoff eingebracht oder in Form einer Fällungskristallisation in einer flüssigen Phase erzeugt werden. Entscheidend sind an dieser Stelle demgegenüber aber die Schmelz- und Rekristallisationsprozesse, die für die hier beschriebenen einfach herstellbaren Harnstoffderivate zu überragenden Eigenschaften als hydrophober Festkörper in Entschäumern führen.

Hierzu muß die Rekristallisation der aufgeschmolzenen Harnstoffderivate zur Erzielung optimaler Wirksamkeit aus einer klaren, homogen im Trägermedium verteilten Schmelze heraus erfolgen. Dies ist in einfacher Weise durch Variation der wesentlichen Parameter, wie der chemischen Natur des Trägeröls, des Harnstoffderivates und der Temperatur, möglich.

Werden die Harnstoffderivate im Trägermedium lediglich aufgeschmolzen und bilden dabei eine inhomogene, das heißt keine klare Mischung, können mit dem erfindungsgemäßen Verfahren keine ausreichend wirksamen Entschäumer hergestellt werden.

Die praktische Durchführung der beschriebenen Schmelz- und Rekristallisationsprozesse kann nach verschiedenen Methoden durchgeführt werden. Beispielsweise kann durch Hinzugabe von oder Hineingabe in ein Trägermedium A definierter Temperatur die Kristallisation des in einem Trägermedium B aufgeschmolzenen Harnstoffderivates je nach Wahl der Temperaturgradienten und Art und Maß der eingebrachten Scherenergie in einem weiten Rahmen beeinflußt werden. Trägermedium A und B können dabei identisch oder unterschiedlich sein.

Ebenso können die Schmelzen der Harnstoffderivate auch durch einfaches Stehenlassen bei Raumtemperatur bis hin zur Zuführung externer Kühlung mit Kühlmitteln in bestimmter Weise zur Kristallisation gebracht werden. Auch hier legen die gewählten Umstände die Kristallisationsform der Harnstoffderivate und damit die Wirksamkeit der so erzeugten Entschäumerformulierungen fest. So kann die Rekristallisation der aufgeschmolzenen Harnstoffderivate auch vorteilhaft in Anwesenheit von zusätzlichen Festkörpern, die als Kristallisationskeime dienen können, durchgeführt werden, um bestimmte gewünschte Kristallmorphologien zu erreichen.

Um im Sinne der vorliegenden Erfindung möglichst effektiv wirksame Harnstoffderivate zu erhalten, ist es wichtig, besonders kleine, sphärische Harnstoffkristalle zu bilden. Dies kann gezielt beeinflußt werden, indem zum Beispiel die Konzentration der Harnstoffderivate in der Schmelze variiert wird. Je geringer man die Konzentration der Harnstoffderivate in der Schmelze wählt, um so kleinere Kristalle kann man erzeugen. Außerdem wird die Bildung kleiner Harnstoffderivatkristalle durch einen großen, schnell durchlaufenen Temperaturgradienten begünstigt, wenn man zum Beispiel eine heiße Schmelze schockartig abkühlt, indem man sie in ein deutlich kühleres Trägermedium gießt. Hohe Rührleistungen mit effektiv durchmischenden Rührern vermeiden an dieser Stelle unerwünscht große, dauerhaft bestehende Konzentrationsgradienten, die wiederum zu größeren Kristallen beziehungsweise deren Agglomeraten führen. Es ist jedoch auch vorteilhaft möglich, zumindest partiell zunächst Bedingungen einzustellen, die die Bildung kleinerer Kristalle bevorzugen sollten und diesen dann Bedingungen folgen zu lassen, die große Kristalle bevorzugen. Beispielsweise kann man eine Schmelze zunächst schnell und erst dann langsam abkühlen. So wird erreicht, daß auf vielen schlagartig gebildeten Kristallisationskeimen dann langsamer gleichmäßig geformte Kristalle aufwachsen.

Als geeignete Trägermedien können neben organischen oder mineralischen Ölen auch Siloxane oder organomodifizierte Siloxane dienen.

Durch Zusatz von Emulgatoren können solche Entschäumer auch in wäßrige Emulsionen überführt werden.

Die erfindungsgemäßen Entschäumer können beispielsweise zur Entschäumung von Kühlschmierstoffen, Polymerdispersionen, Lakken und Druckfarben eingesetzt werden.

### Herstellungsbeispiele:

### Beispiel 1

475 g eines naphthenbasischen Mineralöls (40 mPas/25 °C) werden in einem Gefäß mit Rührvorrichtung bei Raumtemperatur vorgelegt, mit 14,36 g Benzylamin versetzt und 5 Minuten verrührt. Nach Zugabe von 10,64 g Hexamethylendiisocyanat unter Rühren beobachtet man die schlagartige Bildung eines farblosen Niederschlages. Die Nachreaktionszeit beträgt 60 Minuten.

### Beispiel 2

475 g eines Polyethersiloxanes (400 mPas/25 °C; Brechungsindex n=1.440/25 °C; unlöslich in Wasser) werden in einem Gefäß mit Rührvorrichtung bei Raumtemperatur vorgelegt, mit 14,36 g Benzylamin versetzt und 5 Minuten verrührt. Nach Zugabe von 10,64 g Hexamethylendiisocyanat unter Rühren beobachtet man die schlagartige Bildung eines farblosen Niederschlages. Die Nachreaktionszeit beträgt 60 Minuten.

### Beispiel 3

475 g eines naphthenbasischen Mineralöls (40 mPas/25 °C) werden in einem Gefäß mit Rührvorrichtung bei Raumtemperatur vorgelegt, mit 2,78 g Diaminopropan versetzt und 5 Minuten verrührt. Nach Zugabe von 22,22 g Octadecylisocyanat unter Rühren beobachtet man die schlagartige Bildung eines farblosen Niederschlages. Die Nachreaktionszeit beträgt 60 Minuten.

### Beispiel 4

475 g eines Polyethersiloxanes (400 mPas/25 °C; Brechungsindex n=1.440/25 °C; unlöslich in Wasser) werden in einem Gefäß mit Rührvorrichtung bei Raumtemperatur vorgelegt, mit 2,78 g Diaminopropan versetzt und 5 Minuten verrührt. Nach Zugabe von 22,22 g Octadecylisocyanat unter Rühren beobachtet man die schlagartige Bildung eines farblosen Niederschlages. Die Nachreaktionszeit beträgt 60 Minuten.

### Beispiel 5 (erfindungsgemäß)

250 g der in Beispiel 1 hergestellten Mischung werden auf 170 °C erhitzt wobei sich eine klare, homogene Mischung bildet. Diese heiße Mischung wird nun in 250 g kaltes, naphthenbasisches Mineralöl (40 mPas/25 °C) eingeleitet und damit verrührt. Man beobachtet augenblicklich die Bildung eines fein verteilten, farblosen Niederschlages und ein schlagartiges Anziehen der Viskosität.

### Beispiel 6 (erfindungsgemäß)

250 g der in Beispiel 2 hergestellten Mischung werden auf 170 °C erhitzt wobei sich eine klare, homogene Mischung bildet. Diese heiße Mischung wird nun in 250 g kaltes Polyethersiloxan (400 mPas/25 °C; Brechungsindex n=1.440/25 °C; unlöslich in Wasser) eingeleitet und damit verrührt. Man beobachtet augenblicklich die Bildung eines fein verteilten, farblosen Niederschlages und ein schlagartiges Anziehen der Viskosität.

### Beispiel 7 (erfindungsgemäß)

250 g der in Beispiel 3 hergestellten Mischung werden auf 170 °C erhitzt wobei sich eine klare, homogene Mischung bildet. Diese heiße Mischung wird nun in 250 g kaltes, naphthenbasisches Mineralöl (40 mPas/25 °C) eingeleitet und damit verrührt. Man beobachtet augenblicklich die Bildung eines fein verteilten, farblosen Niederschlages und ein schlagartiges Anziehen der Viskosität.

### Beispiel 8 (erfindungsgemäß)

250 g der in Beispiel 4 hergestellten Mischung werden auf 170 °C erhitzt wobei sich eine klare, homogene Mischung bildet. Diese heiße Mischung wird nun in 250 g kaltes _{P}olyethersiloxan (400 mPas/25 °C; Brechungsindex n=1.440/25 °C; unlöslich in Wasser) eingeleitet und damit verrührt. Man beobachtet augenblicklich die Bildung eines fein verteilten, farblosen Niederschlages und ein schlagartiges Anziehen der Viskosität.

### Beispiel 9 (erfindungsgemäß)

225 g naphthenbasisches Mineralöl (40 mPas/25 °C) werden mit 25 g N'N''-Propan-1,3-diylbis(N'-octadecylharnstoff) vermischt und auf 170 °C erhitzt wobei sich eine klare, homogene Mischung bildet. Diese heiße Mischung wird nun in 250 g kaltes, naphthenbasisches Mineralöl (40 mPas/25 °C) eingeleitet und damit verrührt. Man beobachtet augenblicklich die Bildung eines fein verteilten, farblosen Niederschlages und ein schlagartiges Anziehen der Viskosität.

### Beispiel 10 (erfindungsgemäß)

250 g der in Beispiel 1 hergestellten Mischung werden auf 170 °C erhitzt wobei sich eine klare, homogene Mischung bildet. Diese heiße Mischung wird nun in eine Mischung aus 225 g kaltem, naphthenbasischem Mineralöl (40 mPas/25 °C) und 25 g Sorbitantrioleat-20 EO eingeleitet und damit verrührt. Man beobachtet augenblicklich die Bildung eines fein verteilten, farblosen Niederschlages und ein schlagartiges Anziehen der Viskosität.

### Beispiel 11 (erfindungsgemäß)

250 g der in Beispiel 2 hergestellten Mischung werden auf 170 °C erhitzt wobei sich eine klare, homogene Mischung bildet. Diese heiße Mischung wird nun in eine Mischung aus 225 g kaltem Polyethersiloxan (400 mPas/25 °C; Brechungsindex n=1.440/25 °C; unlöslich in Wasser) und 25 g Sorbitantrioleat-20 EO eingeleitet und damit verrührt. Man beobachtet augenblicklich die Bildung eines fein verteilten, farblosen Niederschlages und ein schlagartiges Anziehen der Viskosität.

### Beispiel 12 (nicht erfindungsgemäß)

250 g der in Beispiel 1 hergestellten Mischung werden mit 250 g naphthenbasischem Mineralöl (40 mPas/25 °C) verrührt.

### Beispiel 13 (nicht erfindungsgemäß)

250 g der in Beispiel 2 hergestellten Mischung werden mit 250 g Polyethersiloxan (400 mPas/25 °C; Brechungsindex n=1.440/25 °C; unlöslich in Wasser) verrührt.

### Beispiel 14 (nicht erfindungsgemäß)

250 g der in Beispiel 3 hergestellten Mischung werden mit 250 g naphthenbasischem Mineralöl (40 mPas/25 °C) verrührt.

### Beispiel 15 ( nicht erfindungsgemäß)

250 g der in Beispiel 4 hergestellten Mischung werden mit 250 g Polyethersiloxan (400 mPas/25 °C; Brechungsindex n=1.440/25 °C; unlöslich in Wasser) verrührt.

### Beispiel 16 (nicht erfindungsgemäß)

250 g der in Beispiel 1 hergestellten Mischung werden mit einer Mischung aus 225 g naphthenbasischem Mineralöl (40 mPas/25 °C) und 25 g Sorbitantrioleat-20 EO verrührt.

### Beispiel 17 (nicht erfindungsgemäß)

250 g der in Beispiel 2 hergestellten Mischung werden mit 225 g Polyethersiloxan (400 mPas/25 °C; Brechungsindex n=1.440/25 °C; unlöslich in Wasser) und 25g Sorbitantrioleat-20 EO verrührt.

### Beispiel 18 (nicht erfindungsgemäß)

In einem Rührgefäß werden 130 g eines naphthenbasischen Mineralöls (40 mPas/25 °C) auf 170 °C erhitzt und mit 1,82 g Octylamin versetzt und so lange gerührt, bis das Amin vollständig gelöst war. Nach Zugabe von 1,18 g 1,6-Hexamethylendiisocyanat und einer Nachreaktionszeit von 30 Minuten wird das nicht klare, inhomogene Reaktionsgemisch in einem zweiten Rührgefäß unter intensivem Rühren und Kühlung in 143 g auf 20 °C abgekühlten, naphthenbasischen Mineralöls (40 mPas/25 °C) eingetropft. Man beobachtet augenblicklich die Bildung eines klumpigen, flockigen, farblosen Niederschlages.

### Beispiel 19 (nicht erfindungsgemäß)

In einem Rührgefäß werden 130 g eines naphthenbasischen Mineralöls (40 mPas/25 °C) auf 190 °C erhitzt und mit 1,82 g Octylamin versetzt und so lange gerührt, bis das Amin vollständig gelöst war. Nach Zugabe von 1,18 g 1,6-Hexamethylendiisocyanat und einer Nachreaktionszeit von 30 Minuten wird das nicht klare, inhomogene Reaktionsgemisch in einem zweiten Rührgefäß unter intensivem Rühren und Kühlung in 143 g, auf 20 °C abgekühlten, naphthenbasischen Mineralöls (40 mPas/25 °C) eingetropft. Man beobachtet augenblicklich die Bildung eines klumpigen, flockigen, farblosen Niederschlages.

### Anwendungsbeispiele

Die Überprüfung der anwendungstechnischen Eigenschaften erfolgte in einem handelsüblichen, Mineralöl enthaltenden Kühlschmierstoffkonzentrat und zwei kommerziell erhältlichen Polymerdispersionen.

### Prüfung der entschäumenden Wirkung in einem kommerziell erhältlichen Kühlschmierstoffkonzentrat

99,6 g eines handelsüblichen, Mineralöl enthaltenden Kühlschmierstoffkonzentrates wurden mit 0,4 g der nach Beispiel 5 bis 9 und Beispiel 12 bis 15, 18 und 19 hergestellten Entschäumer versetzt. Davon werden 15 g in einen graduierten 1000 ml Meßzylinder gegeben und mit entionisiertem Wasser auf 300 ml aufgefüllt. Diese Lösung wird nun mittels einer D1-Fritte mit einer Luftgeschwindigkeit von 1,8 l/Minute begast. Es wird die Zeit in Sekunden gemessen, welche benötigt wird, um 700 ml Schaum zu bilden.

**Tabelle 1**

| Ergebnisse der anwendungstechnischen Prüfung in einem Kühlschmierstoffkonzentrat | |
|---|---|
| Zugesetzter Entschäumer | Zeit bis 700 ml Schaum gebildet sind |
| ohne Zusatz | 22 Sekunden |
| Beispiel 5 (erfindungsgemäß) | 2100 Sekunden |
| Beispiel 6 (erfindungsgemäß) | 5220 Sekunden |
| Beispiel 7 (erfindungsgemäß) | 1560 Sekunden |
| Beispiel 8 (erfindungsgemäß) | 3840 Sekunden |
| Beispiel 9 (erfindungsgemäß) | 1980 Sekunden |
| Beispiel 12 (nicht erfindungsgemäß) | 480 Sekunden |
| Beispiel 13 (nicht erfindungsgemäß) | 780 Sekunden |
| Beispiel 14 (nicht erfindungsgemäß) | 180 Sekunden |
| Beispiel 15 (nicht erfindungsgemäß) | 360 Sekunden |
| Beispiel 18 (nicht erfindungsgemäß) | 110 Sekunden |
| Beispiel 19 (nicht erfindungsgemäß) | 90 Sekunden |

Wie den angegebenen Beispielen zu entnehmen ist, sind die erfindungsgemäßen Beispiele (Beispiel 5 bis 9) mit Bezug auf ihre entschäumende Wirkung den nicht erfindungsgemäßen Beispielen (Beispiel 12 bis 15, 18 und 19) deutlich überlegen. Insbesondere wird deutlich, daß zur Erzielung optimaler Wirksamkeit der Entschäumerformulierungen eine Rekristallisation aufgeschmolzener Harnstoffderivate aus einer klaren, homogen im Trägermedium verteilten Schmelze heraus erfolgen muß.

### Prüfung der entschäumenden Wirkung in kommerziell erhältlichen Polymerdispersionen

Die Prüfung erfolgte in der Styrolacrylatdispersion Acronal® 290 D, Fa. BASF und der Reinacrylatdispersion Acronal® A603, Fa. BASF.

Die erfindungsgemäßen und nicht erfindungsgemäßen Entschäumer wurden je eine Minute bei 1000 U/min in die Dispersionen eingearbeitet.

In die mit Entschäumer additivierten Polymerdispersionen wurde 1 Minute mit 2500 U/min mittels eines Turbinenrührers (Durchmesser 4 cm) Luft eindispergiert. Die so bearbeitete Dispersion wurde dann, unmittelbar nach dem Abstellen des Rührers, in eine Mensur bis zur Eichmarke von 50 ml gefüllt und gewogen. Das Gewicht wird durch die eingerührte Luftmenge beeinflußt und ist ein Maß für die Wirksamkeit des Entschäumers.

**Tabelle 2**

| Ergebnisse der anwendungstechnischen Prüfung in der Polymerdispersion Acronal® 290 D | | |
|---|---|---|
| Entschäumer | Menge Entschäumer in Gew.-% | Probendichte in g/50 ml |
| ohne Zusatz | - | 39,3 |
| Beispiel 10 (erfindungsgemäß) | 0,2 | 49,6 |
| Beispiel 11 (erfindungsgemäß) | 0,05 | 50,0 |
| Beispiel 16 (nicht erfindungsgemäß) | 0,2 | 43,6 |
| Beispiel 17 (nicht erfindungsgemäß) | 0,05 | 44,1 |

**Tabelle 3**

| Ergebnisse der anwendungstechnischen Prüfung in der Polymerdispersion Acronal® A603 | | |
|---|---|---|
| Entschäumer | Menge Entschäumer in Gew.-% | Probendichte in g/50 ml |
| ohne Zusatz | - | 38,1 |
| Beispiel 10 (erfindungsgemäß) | 0,2 | 48,8 |
| Beispiel 11 (erfindungsgemäß) | 0,05 | 49,2 |
| Beispiel 16 (nicht erfindungsgemäß) | 0,2 | 42,5 |
| Beispiel 17 (nicht erfindungsgemäß) | 0,05 | 43,9 |

Wie den angegebenen Beispielen zu entnehmen ist, sind die erfindungsgemäßen Beispiele (Beispiel 10 und 11) mit Bezug auf ihre entschäumende Wirkung den nicht erfindungsgemäßen Beispielen (Beispiel 16 und 17) deutlich überlegen.

## Patentansprüche

1. Mittel zur Entschäumung wäßriger Medien, die als Entschäumer Harnstoffderivate der Formel I 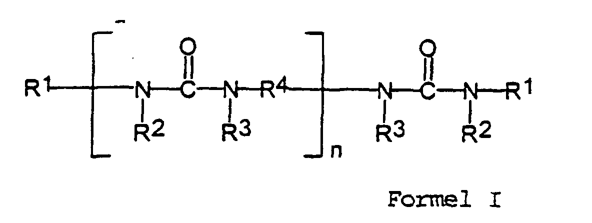 wobei
R¹ - ein Kohlenwasserstoffrest mit 4 bis 30 Kohlenstoffatomen oder ein Kohlenwasserstoffrest mit 4 bis 24 Kohlenstoffatomen und einem Stickstoffatom oder ein Kohlenwasserstoffrest mit 4 bis 30 Kohlenstoffatomen und einer Carbonylgruppe,
R² - ein Wasserstoffatom oder ein Kohlenwasserstoffrest mit 1 bis 24 Kohlenstoffatomen,
R³ - ein Wasserstoffatom oder ein Kohlenwasserstoffrest mit 1 bis 24 Kohlenstoffatomen,
R⁴ - ein organischer Rest mit 2 bis 30 Kohlenstoffatomen und
n - 0 bis 5 sind,
in Form von Feststoffpartikeln enthalten, welche durch Kristallisation aus einer in einem Trägermedium verteilten klaren, homogenen Schmelze heraus erhalten werden.

2. Mittel nach Anspruch 1, **dadurch gekennzeichnet, daß** R¹ ein Kohlenwasserstoffrest mit 4 bis 24 Kohlenstoffatomen ist, R² ein Wasserstoffatom, R³ ein Wasserstoffatom und R⁴ ein Kohlenwasserstoffrest mit 2 bis 24 Kohlenstoffatomen sind.

3. Mittel nach den Ansprüchen 1 oder 2 **dadurch gekennzeichnet, daß** als Trägermedien organische oder mineralische Öle dienen.

4. Mittel nach den Ansprüchen 1 bis 3 **dadurch gekennzeichnet, daß** als Trägermedien Siloxane oder organomodifizierte Siloxane dienen.

5. Die Verwendung von Entschäumern nach den Ansprüchen 1 bis 4 zur Entschäumung von Kühlschmierstoffen.

6. Die Verwendung von Entschäumern nach den Ansprüchen 1 bis 4 zur Entschäumung von Polymerdispersionen.

7. Die Verwendung von Entschäumern nach den Ansprüchen 1 bis 4 zur Entschäumung von Lacken und Druckfarben.

## Claims

1. Composition for defoaming aqueous media, comprising as defoamers urea derivatives of the formula I 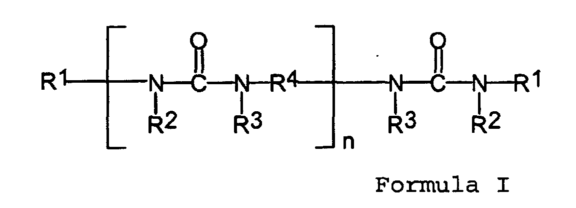 where
R¹ - is a hydrocarbon radical having 4 to 30 carbon atoms or a hydrocarbon radical having 4 to 24 carbon atoms and one nitrogen atom or a hydrocarbon radical having 4 to 30 carbon atoms and one carbonyl group,
R² - is a hydrogen atom or a hydrocarbon radical having 1 to 24 carbon atoms,
R³ - is a hydrogen atom or a hydrocarbon radical having 1 to 24 carbon atoms,
R⁴ - is an organic radical having 2 to 30 carbon atoms, and
n - is from 0 to 5,
in the form of solid particles, which are obtained by crystallization from a clear homogeneous melt dispersed in a carrier medium.

2. Composition according to Claim 1, **characterized in that** R¹ is a hydrocarbon radical having 4 to 24 carbon atoms, R² is a hydrogen atom, R³ is a hydrogen atom, and R⁴ is a hydrocarbon radical having 2 to 24 carbon atoms.

3. Composition according to Claim 1 or 2, **characterized in that** organic or mineral oils are used as carrier media.

4. Composition according to any of Claims 1 to 3, **characterized in that** siloxanes or organomodified siloxanes are used as carrier media.

5. Use of defoamers according to any of Claims 1 to 4 to defoam cooling lubricants.

6. Use of defoamers according to any of Claims 1 to 4 to defoam polymer dispersions.

7. Use of defoamers according to any of Claims 1 to 4 to defoam coating materials and printing inks.

## Revendications

1. Agent anti-mousse pour milieux aqueux, qui contiennent, en tant qu'anti-mousse, des dérivés d'urée de formule I 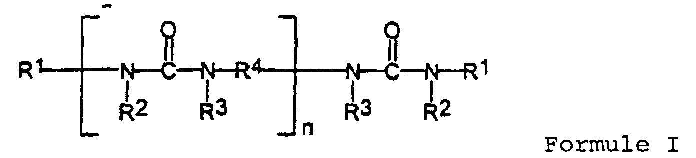 où
R¹ représente un radical hydrocarboné comprenant 4 à 30 atomes de carbone ou un radical hydrocarboné comprenant 4 à 24 atomes de carbone et un atome d'azote ou un radical hydrocarboné comprenant 4 à 30 atomes de carbone et un groupe carbonyle,
R² représente un atome d'hydrogène ou un radical hydrocarboné comprenant 1 à 24 atomes de carbone,
R³ représente un atome d'hydrogène ou un radical hydrocarboné comprenant 1 à 24 atomes de carbone,
R⁴ représente un radical organique comprenant 2 à 30 atomes de carbone et
n vaut 0 à 5,
sous forme de particules solides qui sont obtenues par cristallisation à partir d'une masse fondue homogène claire répartie dans un milieu support.

2. Agent selon la revendication 1, **caractérisé en ce que** R¹ représente un radical hydrocarboné comprenant 4 à 24 atomes de carbone, R² représente un atome d'hydrogène, R³ représente un atome d'hydrogène et R⁴ représente un radical hydrocarboné comprenant 2 à 24 atomes de carbone.

3. Agent selon les revendications 1 ou 2, **caractérisé en ce que** des huiles organiques ou minérales servent de milieu support.

4. Agent selon les revendications 1 à 3, **caractérisé en ce que** des siloxanes ou des siloxanes organiquement modifiés servent de milieu support.

5. Utilisation d'anti-mousses selon les revendications 1 à 4 comme agent anti-mousse de lubrifiants à froid.

6. Utilisation d'anti-mousses selon les revendications 1 à 4 comme agent anti-mousse de dispersions de polymères.

7. Utilisation d'anti-mousses selon les revendications 1 à 4 comme agent anti-mousse de laques et d'encres d'imprimerie.
